# EUROPÄISCHE PATENTSCHRIFT

(11) **EP 0 696 906 B1**
(45) Veröffentlichungstag und Bekanntmachung des Hinweises auf die Patenterteilung: **16.04.1997**
(21) Anmeldenummer: 94916177.2
(22) Anmeldetag: 29.04.1994
(51) Int. Cl.: A61F 2/06, A61N 5/10

(54) **GEFÄSSIMPLANTAT**
VASCULAR IMPLANT
IMPLANT VASCULAIRE

(30) Priorität: 06.05.1993 DE 4315002
(43) Veröffentlichungstag der Anmeldung: 21.02.1996
(73) Patentinhaber: Forschungszentrum Karlsruhe GmbH, 76133 Karlsruhe (DE)
(72) Erfinder: HEHRLEIN, Christoph, D-69118 Heidelberg (DE); FEHSENFELD, Peter, D-76297 Stutensee (DE)
(74) Vertreter: Rückert, Friedrich, Dr.
(86) Internationale Anmeldenummer: EP9401373
(87) Internationale Veröffentlichungsnummer: WO9426205

(56) Entgegenhaltungen:
- EP-A- 0 497 495
- EP-A- 0 539 165
- WO-A-92/03179
- WO-A-93/04735
- US-A- 3 750 653
- US-A- 4 706 652
- US-A- 5 199 939

## Beschreibung

Die Erfindung betrifft die Verwendung von mindestens zwei Radionuklid-Spezies, von denen die eine Spezies eine Halbwertszeit zwischen 7 Stunden und 7 Tagen und die andere Spezies eine Halbwertszeit von mehr als 100 Tagen aufweist, zur Herstellung eines solchen Gefäßimplantats zur Verhinderung und/oder Beseitigung von Gefäßverengungen, das eine nachfolgende Intimahyperplasie verhindert.

Gefäßimplantate, z. B. sogenannte Stents werden in der Medizin eingesetzt, um Gefäßverengungen zu verhindern oder zu beseitigen. Die Gefäßimplantate können in verengte Gefäße eingesetzt und damit das verengte Gefäß aufgeweitet werden. Die Gefäßimplantate weisen beispielsweise die Form von netzartigen Röhren oder von Schraubenfedern auf.

Die bisherigen Erfahrungen mit der Implantation von solchen Gefäßimplantaten zeigen, daß eine überschießende Wucherung der benachbarten Zellen zur erneuten Einengung des Gefäßes z. B. an den Enden des Implantats und damit zur verminderten Wirkung des Implantates führt. Wenn zur Beseitigung einer arteriosklerotischen Stenose (Einengung) ein Gefäßimplantat in menschliche Arterien eingesetzt wird, kommt es innerhalb eines Jahres zur Intimahyperplasie im Bereich der Enden des Gefäßimplantats, die zur Re-Stenose führt. Bei der Infiltration von Gefäßsystemen mit Karzinomzellen, z. B. im Gallengang beim Gallengangskarzinom, erfolgt rasch eine zelluläre Überwucherung des Gefäßimplantats mit Tumorzellen.

Aus der EP 0 433 011 A1 ist ein radioaktives Gefäßimplantat Art bekannt. Mit einem solchen Gefäßimplantat kann die Zellproliferation im Gewebe in den ersten Monaten nach der Implantation wirksam vermindert werden.

Das bekannte Gefäßimplantat besteht vorzugsweise aus einem Metall, dem z. B. durch Zulegieren eines in ein Radionuklid umwandelbaren Isotops und anschließender Bestrahlung oder durch Aufplattieren der Radionuklid-Species auf einen Kern Radioaktivität verliehen wird. Die Radionuklid-Species soll eine Halbwertszeit (t_{½}) im Bereich zwischen 7 Stunden (h) und 100 Tagen (d) aufweisen. Als bevorzugte Radionuklid-Species werden Phosphor-32 (t_{½} = 14,3 d), Vanadium-48 (t_{½} = 16 d) und Gold-198 (t_{½} = 2,7 d) angegeben. Diese Radionuklidspecies zerfallen unter Aussendung von β-Strahlung in die stabilen Tochternuklide Schwefel-32, Titan-48 und Quecksilber-198, so daß die Radioaktivität spätestens (im Fall von ⁴⁸V mit der längsten Halbwertszeit) nach ca. 5,5 Monaten (10 Halbwertszeiten) praktisch vollständig abgeklungen ist.

In der EP 0 539 165 A1 wird vorgeschlagen, ein solches radioaktives Gefäßimplantat in den Gallengang einzusetzen, um das Wachstum maligner Zellen im Gallengang zu verhindern. Für diesen Zweck soll die Halbwertszeit des Isotops vorzugsweise zwischen 10 Stunden und 1000 Tagen liegen.

Es hat sich gezeigt, daß nach dem Abklingen der Radioaktivität auch nach längeren Zeiträumen (bis zu ca. 2 Jahre und mehr) mit einer Zellproliferation und damit einer Intimahyperplasie und einer Re-Stenose zu rechnen ist. Dem könnte zwar dadurch begegnet werden, daß man gemäß der zitierten Druckschrift längerlebige als die bevorzugten Radionuklid-Species einsetzt, z. B. solche Radionuklide, die eine Halbwertszeit bis zu 100 Tagen aufweisen. Gefäßstützen mit solchen Radionukliden würden den Patienten jedoch einer beträchtlichen, nicht mehr tolerierbaren radioaktiven Strahlung aussetzen, da die zu Beginn vorhandene Radioaktivität in der ersten Zeit nach der Implantation nur langsam abklingt. Zudem wäre eine solche radioaktive Strahlung zeitlich ungünstig verteilt. Es hat sich herausgestellt, daß zur Vermeidung einer Intimahyperplasie und einer Re-Stenose sofort nach der Implantation eine verhältnismäßig hohe, mittel- und langfristig jedoch nur eine sehr geringe radioaktive Strahlung erforderlich ist. Einen solchen zeitlichen Verlauf kann man mit Radionukliden einer mittleren Halbwertszeit nicht erreichen.

Zur Herstellung eines radioaktiven Gefäßimplantats aus einem Metall wird angegeben, daß dem Metall ein solches "Element" zugemischt wird, das in ein Radioisotop umgewandelt werden kann. Daneben werden Implantate mit einer radioaktiven Ummantelung beschrieben. Nähere Informationen über das Herstellungsverfahren enthält die Druckschrift nicht. Bei Implantaten mit einer radioaktiven Ummantelung besteht die Gefahr, daß sich die Ummantelung im Körper des Patienten ablöst und verteilt wird.

Aus der WO-A-92/03179 ist ein Implantat zum Einsetzen in Tumorgewebe bekannt. Das Implantat weist beispielsweise die Form einer mehrschichtigen Kapsel oder eines mehrschichtigen Bandes auf und kann sowohl Radionuklide mit kurzer als auch Radionuklide mit langer Halbwertszeit enthalten.

Der Erfindung liegt die Aufgabe zugrunde, diese Nachteile zu beseitigen. Es soll die Verwendung solcher Radionuklid-Spezies vorgeschlagen werden, die das benachbarte Gewebe während des gesamten Zeitraums, in dem eine Re-Stenose zu befürchten ist, einer radioaktiven Strahlung aussetzen, die nach der Implantation kurzfristig relativ hohe Werte erreicht, mittel- und langfristig jedoch gering ist.

Die Aufgabe wird erfindungsgemäß durch die Verwendung gemäß dem ersten und siebten Patentanspruch gelöst. In den abhängigen Ansprüchen werden bevorzugte Weiterbildungen der Erfindung beschrieben.

Ein wesentliches Merkmal der Erfindung ist, daß nach der Implantation des Gefäßimplantats mindestens zwei verschiedene Radionuklid-Species wirksam werden, und zwar eine mit kurzer Halbwertszeit (7 Stunden bis 7 Tage) und eine mit langer Halbwertzeit (mehr als 100 Tage). Die kurzlebige Radionuklid-Species muß bei der Implantation bereits vorhanden sein. Die langlebige Radionuklid-Species kann zusammen mit der kurzlebigen Species vor der Implantation im Gefäßimplantat erzeugt werden; bevorzugt wird jedoch die kurzlebige Radionuklid-Species so gewählt, daß sie direkt oder indirekt in die langlebige Radionuklid-Species zerfällt.

Die Art und die Konzentration der beiden Radionuklid-Species wird vorzugsweise so gewählt, daß innerhalb der ersten drei Tage nach Implantation des Gefäßimplantats mindestens 40 % der Gesamtaktivität freigesetzt werden. Die Gesamtaktivität soll vorzugsweise im Bereich zwischen 3 und 0,3 MBq liegen.

Als Radionuklid-Species mit kurzer Halbwertszeit sind insbesondere Mangan-52 (t_{½} = 5,7 d), Kobalt-55 (t_{½} = 0,73 d), Technetium-96 (t_{½} = 4,3 d), Molybdän-99 (t_{½} = 66 h) und Nickel-57 (t_{½} = 36 h) geeignet. Einsetzbar ist weiterhin Tantal-183 (t_{½} - 5 d) und Rhenium-182 (t_{½} = 13 h). Werden sowohl die kurzlebige Radionuklidspecies als auch die langlebige Species mit der Halbwertszeit von mehr als 100 Tagen vor der Implantation erzeugt, können als langlebige Species die Nuklide Kobalt-57 (t_{½} = 272 d), Eisen-55 (t_{½} = 2,7 a) und Zink-65 (t_{½} = 244 d) eingesetzt werden.

Wie erwähnt, wird die kurzlebige Radionuklid-Species vorzugsweise so gewählt, daß sie direkt oder indirekt in ein Radionuklid mit einer Halbwertszeit von mehr als 100 Tagen zerfällt. Solche kurzlebigen Species sind das Kobalt-55, das in das Tochternuklid Eisen-55 übergeht, Rhenium-181 mit einer Halbwertszeit von 20 h, das in Wolfram-181 (t_{½} = 121 d) zerfällt, und Nickel-57, aus dem Kobalt-57 (t_{½} = 271 d) entsteht. Durch die Wahl solcher kurzlebiger Radionuklid-Species vereinfacht sich die Herstellung der Gefäßimplantate beträchtlich, da nur eine Radionuklid-Species durch Bestrahlen hergestellt werden muß.

Besonders bevorzugt wird als kurzlebige Radionuklid-Species Kobalt-55, das in eisenhaltigen Stählen durch Deuteronen-Bestrahlung erzeugt werden kann. Kobalt-55 zerfällt durch Elektroneneinfang unter Aussendung von weicher Röntgenstrahlung in Eisen-55 mit einer Halbwertszeit von 2,7 Jahren.

Da in der Medizin für Implantate ohnehin Nickel/Titanlegierungen und eisen- und/oder nickelhaltige Stähle, insbesondere der Stahl CrNi 316 L eingesetzt werden, lassen sich die erfindungsgemäßen Implantate mit Kobalt-55 als kurzlebige Radionuklid-Species durch Deuteronen-Bestrahlung von kommerziell erhältlichen, Eisen enthaltenden Implantaten herstellen. Die kurzlebige Radionuklid-Species Nickel-57 erhält man durch Protonenbestrahlung von nickelhaltigen Stählen [Ni-58 (p,pn) Ni-57]. Einer besonderen Fertigung der erfindungsgemäßen Implantate aus bestrahlten Werkstoffen bedarf es daher in diesen Fällen nicht.

Eine weitere Herstellungsmöglichkeit besteht darin, Gefäßimplantate aus einer Tantallegierung zu bestrahlen. Wird als kurzlebige Radionuklid-Species Rhenium-181 verwendet, kann die Herstellung von Tantal-Implantaten ausgehen, denn Rhenium-181 läßt sich aus Tantal-181 durch eine (α, 4n) Reaktion erzeugen.

Gefäßimplantate mit den oben genannten Radionukliden, insbesondere mit Kobalt-55 als kurzlebige Radionuklid-Species und dessen Tochternuklid Eisen-55 oder mit dem kurzlebigen Nickel-57 und dessen Tochternuklid Kobalt-57, weisen einen weiteren wichtigen Vorteil auf: Sie zerfallen durch Elektroneneinfang unter Emission von weicher Röntgenstrahlung, die - wie β-Strahlung - im Körpergewebe eine Eindringtiefe von maximal einigen Millimetern erreicht, durch Überzüge von Thrombosen verhinderndem Material auf dem Implantat jedoch nicht so stark abgeschwächt wird wie β-Strahlung.

In manchen Fällen kann es sinnvoll sein, außer den beiden genannten Radionuklid-Species zusätzlich eine weitere Species vorzusehen, die eine Halbwertszeit im mittleren Bereich von 50 bis zu 100 Tagen aufweist. Als solche Species können die Nuklide Kobalt-56 (t_{½} = 78 d), Kobalt-58 (t_{½} = 71 d) oder Chrom51 (t_{½} = 28 d) eingesetzt werden. Mit drei Nukliden kann der zeitliche Verlauf der Dosisleistung besonders gut an die Erfordernisse angepaßt werden. Die Radionuklid-Species mit der mittleren Halbwertszeit kann ebenfalls entweder direkt vor der Implantation erzeugt werden, oder das oder die kurzlebige(n) Nuklid(e) wird (werden) in der Weise ausgewählt, daß ein direkt oder indirekt ein Tochternuklid mit mittlerer Halbwertszeit entsteht.

Die Erfindung wird im folgenden anhand von Beispielen näher erläutert.

### Beispiel 1

### Herstellung eines Gefäßimplantats

Es wurde ein kommerziell erhältlicher, segmentierter Stent aus dem chirurgischen Edelstahl CrNi 316 L bestrahlt. Die Länge des Stents betrug 15 mm, der Außendurchmesser 1,6 mm (expandierbar auf 6 mm) und die Materialdicke 0,076 mm.

Die Aktivierung des Stents wurde im externen Strahl eines Zyklotrons mit 9,2 MeV Deuteronen durchgeführt. Dazu wurde der Stent in eine Miniaturaufnahme auf einer Bewegungsmaschine mit Hilfe der hochpräzisen Positionier- und Justiereinrichtung einer Maschinenbau-Bestrahlungsanlage so im gerichteten Deuteronenstrahl angeordnet und bewegt, daß eine gleichmäßige und homogene Radioaktivität im Stent erzeugt wurde. Die Energie und die Art der Strahlpartikel waren so ausgewählt, daß in dem CrNi-Stahl ein möglichst hoher Anteil von Radionukliden mit Elektroneneinfang-Übergängen (ε) und daraus resultierender weicher Röntgenstrahlung (Kα, β = 5 bis 6 KeV) sowie β⁺-Übergängen induziert wurde. Diese Strahlungsarten sorgen für die gewünschte Nahwirkung (0,1 bis 6 mm) im Umgebungsgewebe des implantierten Stent.

Das erzeugte Radionuklid-Gemisch insgesamt und seine Wirkung auf das den Stent direkt umgebende zelluläre Gewebe gibt die Tabelle 1 wieder.

Die angestrebte hohe Anfangswirkung (48 % der Gesamtaktivität) in den ersten drei Tagen nach Implantation eines solchen Stents wird durch das Co-55 (41%) und Tc-95 (7%) erreicht. Die Nuklide Mn-52, Tc-96, Mo-99 mit einem Aktivitätsanteil von zusammen 24% sorgen in den folgenden 2 bis 3 Wochen für eine ausreichende Wirkung. Eine mittelfristige Wirkung über etwa 250 Tage wird durch die Nuklide Co-56 und Co-58 erzielt. Fe-55 (das Tochternuklid von Co-55) und Co-57 sorgen für eine langfristige (bis ca. 9 Jahre) Reizminderung im Umgebungsgewebe.

### Beispiel 2

### Herstellung eines Gefäßimplantats

Der in Beispiel 1 beschriebene Versuch wurde mit dem gleichen kommerziell erhältlichen Stent unter analogen Bedingungen wiederholt, wobei jedoch mit 30 MeV-Protonen bestrahlt wurde.

Das erzeugte Radionuklid-Gemisch insgesamt und seine Wirkung auf das den Stent direkt umgebende zelluläre Gewebe gibt die Tabelle 2 wieder.

Dieses Radionuklidgemisch zeichnet sich gegenüber dem gemäß Beispiel 1 erhaltenen Nuklidgemisch durch eine um 100 % höhere Wirkung im Nahbereich (0,1 mm bis 6 mm) des den Stent umgebenden Gewebes aus, da die Röntgenstrahlungs- und β⁺-Energien wesentlich geringer sind.

### Beispiel 3

### Tierversuche

Der gemäß Beispiel 1 aktivierte Stent wurde in Tierversuchen in Arterien von New Zealand White Kaninchen implantiert. Die Kaninchen, die ein Gewicht von 2,5 bis 3 kg aufwiesen, wurden narkotisiert; in beide Beinarterien wurde ein Stent implantiert, und zwar in eine Arterie ein nicht aktivierter und in die andere Arterie der gemäß Beispiel 1 aktivierte Stent.

Drei Kaninchen wurden vier Wochen lang und weitere drei Kaninchen wurden 12 Wochen lang nachbeobachtet. Nach vier bzw. 12 Wochen wurden die Tiere geopfert und die Arterien entnommen. Die Arterien wurden mit Formalin fixiert und anschließend in Epon-Harz eingebettet. Mit einer diamantbeschichteten Säge wurden die Gefäße mit den Stents in 50 µm dicke Gefäßquerschnitte gesägt und diese mit Toluidin-Blau gefärbt.

Die gefärbten Gefäßquerschnitte wurden unter dem Lichtmikroskop untersucht und die Ausmaße der Gewebsanteile (Intimahyperplasie, Gewebsproliferation) mit einem Computerprogramm quantifiziert, wobei sechs Querschnitte des aktivierten Stents und weitere sechs Querschnitte von Kontroll-Stents vermessen und die Daten aufsummiert wurden.

Die Mittelwerte und die Standardabweichung der Daten der insgesamt 12 Gefäße mit den Stents ergaben folgende Werte:

| Gewebsproliferation bzw. Intimahyperplasie in [mm²]: | nach 4 Wochen | nach 12 Wochen |
|---|---|---|
| Aktivierte Stents | 0,2 ± 0,03 | 0,1 ± 0,04 |
| Kontroll-Stents | 1,0 ± 0,2 | 0,9 ± 0,1 |

Die Unterschiede sind statistisch signifikant und zeigen deutlich, daß die überschießende und unerwünschte Gewebsproliferation bzw. Intimahyperplasie der Kontroll-Stents durch deren Aktivierung gehemmt werden kann, wobei hervorzuheben ist, daß die wirksame radioaktive Strahlung hierbei im wesentlichen eine weiche Röntgenstrahlung ist. Die Werte für die KontrollStents decken sich mit den Ergebnissen früherer Versuchsreihen.

Blutbildveränderungen durch die Radioaktivität der aktivierten Stents konnten nicht festgestellt werden, d. h., die antiproliferative Wirkung der aktivierten Stents war lokal begrenzt. Das umliegende Gewebe wie z. B. von Blase und Darm blieb unverändert. In den Exkrementen war keine Aktivität nachweisbar. Wegen des Gamma-Anteils der Strahlung ließen sich die aktivierten Stents im Körper gut lokalisieren und bildlich darstellen. Eine Veränderung des Stent-Werkstoffs durch die Radioaktivität bleibt wegen des minimalen Radionuklid-Atomanteils von maximal 10⁻¹⁰ ausgeschlossen.

**Tabelle 1**

| Radionuklid | Erzeugte Aktivität in MBq () Anteil an Gesamtakt. | Halbwertszeit | Wirkungsdauer *) | Wirksame Strahlungsart ε = Elektroneneinfang mit result. Röntgenstrahlung (x); β⁺ = Positronenstrahlung |
|---|---|---|---|---|
| Co-55; | 1,25 (41%) | 17,5 h | 2,5 d | ε 21% x 6,5 KeV |
| | | | | β⁺ 80% <1,5 MeV |
| Tochternuklid Fe-55 | 0,00094 (0,04%) | 2,7 a | 9 a | ε 100% x 6 KeV |
| | | | | β⁺ 0% |
| Mn-52 | 0,5 (16%) | 5,7 d | 20 d | ε 70% x 5,4 KeV |
| | | | | β⁺ 30% <0,6 MeV |
| Co-57 | 0,39 (13%) | 272 d | 2,5 a | ε 100% x 6,4 KeV |
| | | | | β⁺ 0% |
| Co-56 | 0,32 (11%) | 78 d | 260 d | ε 80% x 6,5 KeV |
| | | | | β⁺ 20% <1,5 MeV |
| Tc-95 | 0,2 (7%) | 20 h | 2,8 d | ε 100% x17,5 KeV |
| | | | | β⁺ 0% |
| Tc-96 | 0,13 (4%) | 4,3 d | 14 d | ε 100% x17,5 KeV |
| | | | | β⁺ 0% |
| Co-58 | 0,12 (4%) | 71 d | 235 d | ε 85% x 6,5 KeV |
| | | | | β⁺ 15% <0,5 MeV |
| Mo-99 | 0,12 (4%) | 66 h | 9 d | ε 0% |
| | | | | β⁺100% <1,23 MeV) |

| | | | | |
|---|---|---|---|---|
| *) Als Wirkungsdauer ist die Zerfallszeit bis auf 10% der Anfangsaktivität angegeben. | | | | |

**Tabelle 2**

| Radionuklid | Erzeugte Aktivität in MBq () Anteil an Gesamtakt. | Halbwertszeit | Wirkungsdauer *) | Wirksame Strahlungsart ε = Elektroneneinfang mit result. Röntgenstrahlung (x); β⁺ = Positronenstrahlung |
|---|---|---|---|---|
| Co-55; | 1,25 (58%) | 17,5 h | 2,5 d | ε 21% x 6,5 KeV |
| | | | | β⁺ 80% <1,5 MeV |
| Tochternuklid Fe-55 | 0,00094 (0,04%) | 2,7 a | 9 a | ε 100% x 6 KeV |
| | | | | β⁺ 0% |
| Ni-57 | 0,44 (20%) | 36 h | 5 d | ε 54% x 6,9 KeV |
| | | | | β⁺ 46% <0,8 MeV |
| Cr-51 | 0,20 (9%) | 27,7d | 92 d | ε 100% x 4,9 KeV |
| | | | | β⁺ 0% |
| Mn-52 | 0,12 (6%) | 5,7 d | 20 d | ε 70% x 5,4 KeV |
| | | | | β⁺ 30% <0,6 MeV |
| Tc-95 | 0,06 (3%) | 20 h | 2,8 d | ε 100% x 17,5 KeV |
| | | | | β⁺ 0% |
| Co-56 | 0,05 (2,3%) | 78 d | 260 d | ε 80% x 6,5 KeV |
| | | | | β⁺ 20% <1,5 MeV |
| Tc-96 | 0,02 (0,9%) | 4,3 d | 14 d | ε 100% x 17,5 KeV |
| | | | | β⁺ 0% |
| Co-57 | 0,01 (0,5%) | 271 d | 2,5 a | ε 100% x 6,4 KeV |
| | | | | β⁺ 0% |
| Mo-99 | 0,006 (0,3%) | 66 h | 9 d | ε 0% |
| | | | | β⁺100% <1,23 MeV) |

| | | | | |
|---|---|---|---|---|
| *) Als Wirkungsdauer ist die Zerfallszeit bis auf 10% der Anfangsaktivität angegeben. | | | | |

## Patentansprüche

1. Verwendung von mindestens zwei Radionuklid-Spezies, von denen die eine Spezies eine Halbwertszeit zwischen 7 Stunden und 7 Tagen und die andere Spezies eine Halbwertszeit von mehr als 100 Tagen aufweist, zur Herstellung eines solchen Gefäßimplantats zur Verhinderung und/oder Beseitigung von Gefäßverengungen, das eine nachfolgende Intimahyperplasie verhindert.

2. Verwendung nach Anspruch 1 mit solchen Radionuklid-Spezies, die weiche Röntgenstrahlung emittieren.

3. Verwendung nach Anspruch 1 oder 2 mit Kobalt-55 oder Mangan-52 oder Technetium-96 oder Molybdän-99 oder Nickel-57 als Radionuklid-Spezies mit der Halbwertszeit zwischen 7 Stunden und 7 Tagen.

4. Verwendung nach Anspruch 1, 2 oder 3 mit Eisen-55 oder Zink-65 oder Kobalt-57 als Radionuklid-Species mit der Halbwertszeit von mehr als 100 Tagen.

5. Verwendung nach einem der Ansprüche 1 bis 4 mit einer zusätzlichen Radionuklid-Spezies, die eine Halbwertszeit von 50 bis 100 Tagen aufweist.

6. Verwendung nach Anspruch 5 mit Kobalt-56 oder Kobalt-58 oder Chrom-51 als zusätzliche Radionuklid-Spezies.

7. Verwendung einer Radionuklid-Spezies, die eine Halbwertszeit zwischen 7 Stunden und 7 Tagen aufweist und direkt oder indirekt in mindestens ein Tochternuklid mit einer Halbwertszeit von mehr als 100 Tagen zerfällt, zur Herstellung eines solchen Gefäßimplantats zur Verhinderung und/oder Beseitigung von Gefäßverengungen, das eine nachfolgende Intimahyperplasie verhindert.

8. Verwendung nach Anspruch 7 mit einer Radionuklid-Spezies und einem Tochternuklid, die weiche Röntgenstrahlung emittieren.

9. Verwendung nach Anspruch 7 oder 8 mit Kobalt-55 oder Rhenium-181 oder Nickel-57 als Radionuklid-Spezies.

## Claims

1. Use of at least two radionuclide species, of which one species has a half-life period of between 7 hours and 7 days, and the other species has a half-life period of more than 100 days, to produce such a vascular implant to prevent and/or eliminate vascular constrictions, which implant prevents a subsequent intimahyperplasia.

2. Use according to claim 1 with such radionuclide species which emit soft X-radiation.

3. Use according to claim 1 or 2 with cobalt-55 or manganese-52 or technetium-96 or molybdenum-99 or nickel-57 as the radionuclide species having the half-life period of between 7 hours and 7 days.

4. Use according to claim 1, 2 or 3 with iron-55 or zinc-65 or cobalt-57 as the radionuclide species having the half-life period of more than 100 days.

5. Use according to one of claims 1 to 4 with an additional radionuclide species which has a half-life period of between 50 and 100 days.

6. Use according to claim 5 with cobalt-56 or cobalt-58 or chromium-51 as the additional radonuclide species.

7. Use of a radionuclide species, which has a half-life period of between 7 hours and 7 days and directly or indirectly decomposes into at least one daughter nuclide having a half-life period of more than 100 days, to produce such a vascular implant to prevent and/or eliminate vascular constrictions, which implant prevents a subsequent intimahyperplasia.

8. Use according to claim 7 with a radionuclide species and a daughter nuclide, which emit soft X-radiation.

9. Use according to claim 7 or 8 with cobalt-55 or rhenium-181 or nickel-57 as the radionuclide species.

## Revendications

1. Utilisation d'au moins deux espèces de radionuclide parmi lesquelles une espèce qui possède un temps de demi-vie compris entre 7 heures et 7 jours et l'autre espèces un temps de demi-vie de plus de 100 jours, en vue de la production d'un implant vasculaire, pour empêcher et/ou éliminer les étranglements dans les vaisseaux qui empêchent une hyperplasie subséquente de l'intima.

2. Utilisation selon la revendication 1 avec celles des espèces de radionuclides qui émettent un rayonnement faible.

3. Utilisation selon la revendication 1 ou la revendication 2 avec du cobalt-55 ou du manganèse-52, ou du technetium-96 ou du molybdène-99 ou du nickel-57 comme espèce de radionuclide ayant un temps de demi-vie compris entre 7 heures et 7 jours.

4. Utilisation selon la revendication 1, 2 ou 3, avec du fer-55 ou du zinc-65 ou du cobalt-57 en tant qu'espèce de radionuclide avec un temps de demi-vie de plus de 100 jours.

5. Utilisation selon l'une des revendications 1 à 4, avec un espèce de radionuclide supplémentaire qui possède un temps de demi-vie de 50 à 100 jours.

6. Utilisation selon la revendication 5 avec le cobalt-56 ou le cobalt-58 ou le chrome-51 comme espèce de radionuclide supplémentaire.

7. Utilisation d'une espèce de radionuclide, qui possède un temps de demi-vie compris entre 7 heures et 7 jours et qui se désintègre directement ou indirectement en au moins un nuclide fille ayant un temps de demi-vie de plus de 100 jours, en vue de la production d'un tel implant vasculaire pour empêcher et/ou éliminer les étranglements des vaisseaux qui empêchent une hyperplasie subséquente de l'intima.

8. Utilisation selon la revendication 7 avec une espèce de radionuclide et un nuclide fille qui émettent un rayonnement X faible.

9. Utilisation selon la revendication 7 ou la revendication 8, avec le cobalt-55 ou le rhénium-181 ou le nickel-57 en tant qu'espèce de radionuclide.
